# EUROPÄISCHE PATENTSCHRIFT

(11) **EP 0 825 171 B1**
(45) Veröffentlichungstag und Bekanntmachung des Hinweises auf die Patenterteilung: **16.08.2000**
(21) Anmeldenummer: 97113445.7
(22) Anmeldetag: 04.08.1997
(51) Int. Cl.: C07C 59/01, C07C 51/43, C07C 51/46, C07C 51/285

(54) **Verfahren zur Isolierung von Hydroxypivalinsäure aus wässriger Lösung**
Process for the isolation of hydroxypivalic acid from aqueous solution
Procédé d'isolation d'acide hydroxypivalique à partir d'une solution aqueuse

(30) Priorität: 16.08.1996 DE 19632924
(43) Veröffentlichungstag der Anmeldung: 25.02.1998
(73) Patentinhaber: BAYER AG, 51368 Leverkusen (DE)
(72) Erfinder: Neumann, Karl Heinz, Dr., 53757 Sankt Augustin (DE); Heitkamp, Dieter, Dr., 51399 Burscheid (DE); Fiege, Helmut, Dr., 51373 Leverkusen (DE)

(56) Entgegenhaltungen:
- CHEMICAL ABSTRACTS, vol. 71, no. 7, 18.August 1969 Columbus, Ohio, US; abstract no. 30073j, HUANG, CHING-YUN ET AL.: "2,2-Dimethyl-3-hydroxypropionic acid" Seite 248; Spalte 1; XP002054608 & JP 06 824 888 A (JAPAN GAS-CHEMICAL CO., INC.)

## Beschreibung

Die Erfindung betrifft ein Verfahren zur Isolierung von Hydroxypivalinsäure aus wäßriger Lösung durch azeotrop-destillative Entfernung des Wassers unter vermindertem Druck und unter Einsatz eines hierfür geeigneten organischen Lösungsmittels oder Lösungsmittelgemisches und anschließende Kristallisation der Hydroxypivalinsäure aus einem organischen Lösungsmittelgemisch, welches sich aus einer polaren und einer unpolaren Komponente zusammensetzt und von dem eine oder beide Komponenten bei der vorangegangenen Azeotropdestillation eingesetzt wurden.

Neben der Kaliumpermanganatoxidation von Neopentylglykol und der Cannizzarro-Reaktion von Hydroxypivalaldehyd, die beide aus industrieller Sicht relativ unattraktiv sind, kann Hydroxypivalinsäure durch Luft/O₂-Oxidation von Neopentylglykol oder durch H₂O₂-Oxidation von Hydroxypivalaldehyd hergestellt werden. Die Oxidation von Neopentylglykol mit O₂ oder Luft an einem Pd/C- oder Pt/C-Katalysator in alkalischer wäßriger Lösung zu Hydroxypivalinsäure ist z.B. in JP 53/77 010 oder in US 3 799 977 beschrieben. In beiden Patenten werden keine Vorschriften zur Freisetzung der als Na-Salz anfallenden Hydroxypivalinsäure gegeben. Außerdem fehlen in US 3 799 977 Ausbeute und Selektivitätsangaben völlig und in JP 53/77 010 wird eine sehr zweifelhafte Ausbeute von 100 % angegeben, die lediglich auf dem Vergleich von IR-Banden des nach JP 53/77 010 hergestellten Natriumhydroxypivalats mit einem reinen Natriumhydroxypivalatmuster beruht. Alternativ zu diesem Verfahren kann Hydroxyypivalinsäure auch durch Oxidation von Hydroxypivalaldehyd mit Wasserstoffperoxid synthetisiert werden. Dieses Verfahren wurde in Monatshefte für Chemie 95, (1964), 410 erstmals beschrieben und in einer verbesserten Variante 1968 in JP 43/24 888 nochmals aufgegriffen. Die Hydroxypivalinsäure fällt nach der Wasserstoffperoxidoxidation in wäßriger Lösung an. Zur Isolierung wird die Hydroxypivalinsäure nach Monatshefte durch Zugabe von Na₂CO₃ in das Na-Salz übergeführt. Dies wird durch Zugabe des 10-fachen Volumens Aceton gefällt und abfiltriert. Der Rückstand wird in wenig Wasser gelöst, die Hydroxypivalinsäure mit der stöchiometrischen Menge H₂SO₄ freigesetzt und in CHCl₃ aufgenommen. Die wasserfeuchte CHCl₃-Lösung wird mit Na₂SO₄ getrocknet, das Na₂SO₄ abfiltriert und das CHCl₃ abgedampft. Es bleibt eine Rohhydroxypivalinsäure mit einem Schmelzbereich von 100 bis 122°C (Lit.: 124-126°C).

Dieser große Schmelzbereich zeigt an, daß die Hydroxypivalinsäure eine beträchtliche Menge an mitgeschleppten Verunreinigungen enthält. Nimmt man die Rohhydroxypivalinsäure gemäß Monatshefte als rein an, so beträgt die hypothetische Ausbeute ca. 69 % der theoretischen Ausbeute, bezogen auf den Hydroxypivalaldehydeinsatz. Wirklich saubere Hydroxypivalinsäure mit einem Schmelzpunkt von 125 bis 126°C erhalten die Autoren erst nach weiteren Kristallisationen, zuerst aus CHCl₃ und anschließend aus Wasser. Da die Hydroxypivalinsäure ausgezeichnet wasserlöslich ist, muß davon ausgegangen werden, daß die Ausbeute an reiner Hydroxypivalinsäure nach diesen weiteren Umkristallisationen deutlich unter 50 % der theoretischen Ausbeute absinkt. Die Volumenvergrößerung durch die Acetonzugabe und die große Anzahl an Aufarbeitungsschritten machen dieses Verfahren ebenfalls industriell unattraktiv.

JP 43/24 888 beschreibt nun eine Variante des obigen Verfahrens unter Einsatz eines Katalysators, der in der Lage ist, Wasserstoffperoxid zu zersetzen. Als solche Katalysatoren werden sehr feine Gold-, Platin-, Silber- oder Glasteilchen und auch UV-Licht mit einer Wellenlänge von 200 bis 400 nm (2000 bis 4000 Å) angegeben. In dieser Patentanmeldung wird weiter auf die Schwierigkeiten eingegangen, Hydroxypivalinsäure durch Kristallisation aus wäßriger Lösung zu gewinnen; es konnte aus Wasser kein Material isoliert werden, das einen Schmelzpunkt oberhalb von 120°C hat. Die Autoren entfernen deshalb das Wasser vollständig durch azeotrope Destillation mit einem geeigneten Lösungsmittel, wie Benzol, Toluol, Cyclohexan, n-Butanol u.a.; in den Patentbeispielen wird aber nur Benzol eingesetzt. Nach der Entwässerung wird das Lösungsmittel abgezogen und die rohe Hydroxypivalinsäure fraktionierend unter vermindertem Druck destilliert. Bei einer Nacharbeitung von Beispiel 2 aus JP 43/24 888 mit Toluol statt Benzol (aus arbeitshygienischen Gründen) wurde das Wasser azeotrop abdestilliert. In der anschließenden Hydroxypivalinsäuredestillation konnten von den in der Lösung ursprünglich vorhandenen 2,35 Mol Hydroxypivalinsäure nur 0,64 Mol bei 20 mbar abdestilliert werden. Die bifunktionelle Hydroxypivalinsäure bildet beim Destillieren Polyester und Anhydride, die als dunkelbrauner Destillationsrückstand zurückbleiben. Außerdem muß das Destillat bei ca. 125°C in der Schmelze gehalten werden. Dies kann ebenfalls zu Ausbeute- und Qualitätsverlusten aufgrund von Oligo-/Polymerisierungsreaktionen führen. Anschließend muß das Hydroxypivalinsäuredestillat noch auf einem Band gekühlt und mechanisch zerkleinert werden.

Alternativ wird die Hydroxypivalinsäure nach der azeotropen Entwässerung aus dem Schleppmittel Benzol gemäß JP 43/24 888 durch Abkühlen kristallisiert, muß aber, um die geforderte Qualität zu erreichen, nochmals aus Benzol umkristallisiert werden.

Da Benzol als karcinogen eingestuft ist, gilt die Kristallisation aus diesem Lösungsmittel heute als arbeitshygienisch bedenklich. Eigene Arbeiten zeigen, daß nach der azeotropen Entwässerung eines Oxidaationsansatzes mit dem weniger bedenklichen Toluol die anschließende Kristallisation aus dem Lösungsmittel zu einer klebrigen Hydroxypivalinsäure mit einem Gehalt unter 95 % (GC) führte; dies ist offensichtlich auf die oben erwähnten mitgeschleppten Verunreinigungen zurückzuführen.

Die bisher beschriebenen Verfahren zur Isolierung von Hydroxypivalinsäure sind aufgrund der Anzahl an Aufarbeitungsschritten bzw. der starken Tendenz der Polyester-/Anhydridbildung bei höheren Temperaturen für die industrielle Herstellung von Hydroxypivalinsäure nur wenig geeignet.

Überraschenderweise wurde nun gefunden, daß erstens zur Entfernung des Wassers unter vermindertem Druck gearbeitet werden muß, zweitens die Entfernung des Wassers nicht bis zur völligen Trockenheit durchgeführt werden darf und drittens durch Kombination eines unpolaren organischen Lösungsmittels mit einem polaren organischen Lösungsmittel die Kristallisation der Hydroxypivalinsäure so verbessert werden kann, daß die Hydroxypivalinsäure schon in der 1. Kristallisation mit einer Reinheit von mindestens 98,0 % anfällt. Eine weitere Reinigungsoperation, wie die Destillation der Hydroxypivalinsäure oder die erneute Kristallisation, wird dadurch überflüssig.

Die Erfindung betrifft ein Verfahren zur Isolierung von Hydroxypivalinsäure aus wäßrigen Lösungen durch azeotrop-destillative Entfernung des Wassers und anschließende Kristallisation der Hydroxypivalinsäure aus einem organischen Lösungsmittel, das dadurch gekennzeichnet ist, daß man (a) der wäßrigen Lösung ein oder mehrere organische Lösungsmittel zusetzt, von denen wenigstens eines mit Wasser ein Azeotrop bildet, (b) unter vermindertem Druck soviel Wasser abdestilliert, daß die dabei entstehende Lösung der Hydroxypivalinsäure in dem (den) organischen Lösungsmittel(n) einen Restwassergehalt von mindestens 0,1 Gew.-%, bezogen auf das Gesamtgewicht der entstandenen Lösung, hat und (c) die entstandene Lösung so einstellt, daß sie mindestens ein polares und mindestens ein unpolares Lösungsmittel enthält und die Hydroxypivalinsäure daraus kristallisiert.

Unpolare Lösungsmittel für die Kristallisation sind beispielsweise aliphatische und aromatische C₆-C₁₀-Kohlenwasserstoffe, wie Benzol, Toluol, Xylol, Ethylbenzol, Cyclohexan, n-Hexan, Methylcyclohexan oder ein Gemisch mehrerer von ihnen.

Polare Lösungsmittel für die Kristallisation sind beispielsweise C₁-C₆-Alkohole, Ester mit 3-10 Gesamt-C-Atomen, C₃-C₈-Ketone, C₄-C₁₀-Ether, C₃-C₆-Nitrile oder ein Gemisch mehrerer von ihnen, wie Methanol, Ethanol, n- oder i-Propanol, n- oder i-Butanol, Pentanol, Hexanol, Ethylacetat, Isopropylacetat, i-, n-Butylacetat, 1-Methoxy-2-propylacetat, Methyl-ethylketon, Methyl-isobutylketon, Methyl-tert.-butylether, Diisopropylether, Acetonitril oder ein Gemisch mehrerer von ihnen.

Das zur Kristallisation im erfindungsgemäßen Verfahren eingesetzte Gemisch aus mindestens einem unpolaren und mindestens einem polaren Lösungsmittel enthält wenigstens eines, das mit Wasser ein Azeotrop bildet und das daher bereits in der Azeotropdestillation eingesetzt wird. Azeotropbildende Lösungsmittel finden sich sowohl in der Gruppe der unpolaren als auch der polaren Lösungsmittel. Für den Fall, daß zur Azeotropdestillation ein unpolares Lösungsmittel benutzt wurde, wird zur Einstellung der entstandenen Lösung zur Kristallisation ein polares Lösungsmittel nachgesetzt und umgekehrt. Selbstverständlich ist es möglich, mehrere Lösungsmittel aus jeder Gruppe heranzuziehen; dies ist aber wegen der komplizierten Aufarbeitung weniger bevorzugt. Selbstverständlich ist es auch möglich, zur Azeotropdestillation bereits ein Gemisch aus mindestens einem polaren und mindestens einem unpolaren Lösungsmittel, von denen wenigstens eines mit Wasser ein Azeotrop bildet, einzusetzen und so die Einstellung der entstehenden Lösung zur Kristallisation vorwegzunehmen.

Als besonders geeignet haben sich die beiden Lösungsmittelkombinationen Toluol/1-Butanol und Cyclohexan/n-Butylacetat erwiesen. Hierbei werden zur Kristallisation bevorzugt Lösungsmittelgemische folgender Zusammensetzung eingesetzt: 95 bis 40 Vol.-Teile Toluol und 5 bis 60 Vol.-Teile 1-Butanol oder 80 bis 30 Vol.-Teile n-Butylacetat und 20 bis 70 Vol.-Teile Cyclohexan.

Besonders bevorzugt werden Lösungsmittelkombinationen aus 95 bis 75 Vol.-Teilen Toluol und 5 bis 25 Vol.-Teilen 1-Butanol bzw. 75 bis 50 Vol.-Teilen n-Butylacetat und 25 bis 50 Vol.-Teilen Cyclohexan eingesetzt.

Es ist ein weiteres Kennzeichen, daß die azeotrop-destillative Wasserentfernung unter vermindertem Druck von 10-500 mbar, bevorzugt 30-400 mbar, durchgeführt wird. Selbstverständlich ist es möglich, vor Eintritt in die Azeotropdestillation einen Teil des Wassers, beispielsweise 10-70% der gesamten Wassermenge vor dem Zusatz des Azeotropbildners in einer einfachen Destillation zu entfernen, bei der ebenfalls unter dem angegebenen vermindertem Druck gearbeitet wird.

Die Entfernung des Wassers wird bis zu einem Punkt durchgeführt, bei dem in der entstehenden Lösung der Hydroxypivalinsäure noch mindestens 0,1 Gew.-% Wasser, bezogen auf das Gesamtgewicht der entstandenen Lösung, vorliegt, beispielsweise 0,1-2 Gew.-%, bevorzugt 0,5-1,5 Gew.-% Wasser.

Das Verfahren wird beispielsweise so durchgeführt, daß Hydroxypivalaldehyd, der beispielsweise durch Kondensation von Isobutyraladehyd mit Formaldehyd unter Trialkylaminkatalyse hergestellt wurde, mit H₂O₂ zu Hydroxypivalinsäure oxidiert wird. Alternativ zum Trialkylaminkatalysator können auch andere Basen, wie z.B. Na₂CO₃ (JP 43/24888) oder NaOH bzw. KOH (J. Am. Chem. Soc. 62 (1940), 1785; Chem. Ber. 102 (1969), 1606) in der Aldehydkondensation eingesetzt werden. Die anfallende wäßrige Hydroxypivalinsäurelösung wird mit Mineralsäure beispielsweise auf pH = 2,5, allgemein auf pH = 2-4, gestellt und anschließend etwa die Hälfte der in der Lösung vorhandenen Wassermenge unter vermindertem Druck abdestilliert. Dann gibt man das mit Wasser ein Azeotrop bildende organische Lösungsmittel bzw. Lösungsmittelgemisch zu und entfernt das restliche Wasser durch azeotrope Destillation unter vermindertem Druck bis auf einen Restgehalt von ca. 1 Gew.-% aus der Lösung. Eine Überentwässerung, beispielsweise unter 0,1 Gew.-% Wasser im Destillationssumpf, muß sorgfältig vermieden werden, da sich andernfalls Oligomere und/oder Anhydride der Hydroxypivalinsäure bilden können.

In der Entwässerung kann sowohl das mit Wasser ein Azeotrop bildende Lösungsmittel, wie Toluol, allein als auch das Lösungsmittelgemisch, beispielsweise aus Toluol und 1-Butanol, eingesetzt werden. Das gleiche gilt auch für weitere Kombinationen, beispielsweise aus n-Butylacetat oder Cyclohexan; jede der Komponenten (polar bzw. unpolar) kann allein oder als Lösungsmittelgemisch zur Entwässerung eingesetzt werden. Von bevorzugter Bedeutung für die Qualität der Hydroxypivalinsäure ist, daß die azeotrope Entwässerung unter vermindertem Druck stattfindet und dabei eine Sumpftemperatur von 90°C, besonders bevorzugt von 65°C nicht überschritten wird. Entwässert man bei Normaldruck, so kommt es zu einer Gelbfärbung des Sumpfes, die bis in das kristallisierte Produkt durchschlägt. Zur Kristallisation wird das zweite Lösungsmittel zugegeben, falls es nicht schon zur Entwässerung eingesetzt wurde. Die produkthaltige Lösung wird dann, da in der industriellen Produktion meist Sole als Kühlmittel zur Verfügung steht, bevorzugt auf bis zu -18°C, aus Kostengründen besonders bevorzugt bis zu -8°C abgekühlt. Die Hydroxypivalinsäurekristalle werden abfiltriert, mit dem Lösungsmittelgemisch gewaschen und bei max. 60°C unter vermindertem Druck im Trockenschrank getrocknet.

Die folgenden Beispiele sollen den Gegenstand der Erfindung erläutern, aber in keiner Weise einschränken.

### Beispiel 1 (zum Vergleich)

In einem 4 l-Vierhalskolben mit Rührer, Innenthermometer und Wasserabscheider wurden 1600 g einer aus einer H₂O₂-Oxidation stammenden Hydroxypivalinsäurelösung mit einem Säuregehalt von 36,95 Gew.-% vorgelegt und mit 1 l Toluol versetzt. Die Lösung wurde bei Normaldruck, beginnend bei 85°C Sumpftemperatur bis zu einer Sumpftemperatur von 110°C, azeotrop entwässert. Während der Entwässerung trat eine gelbliche Verfärbung der Reaktionslösung auf. Der Restwassergehalt lag bei ∼1,3 Gew.-% nach Karl-Fischer-Titration. Die Lösung wurde erst schnell unter Rühren bis zur beginnenden Kristallisation bei 40°C gekühlt und dann mit 10°C/h bis auf 0°C weiter abgekühlt. Die Hydroxypivalinsäurekristalle wurden abgesaugt, mit 200 ml Toluol gewaschen und bei 150 mbar/55°C im Trockenschrank getrocknet. Man erhielt 503,9 g Hydroxypivalinsäure mit einem Gehalt von 91,31 % (GC int. Std.) 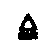 77,8 % der in der Einsatzlösung vorhandenen Hydroxypivalinsäure. Dieses Beispiel zeigt, daß die azeotrope Entwässerung mit Toluol allein bei Normaldruck und anschließender Kristallisation zu einer unbefriedigenden Hydroxypivalinsäurequalität führt.

### Beispiel 2 (zum Vergleich)

In der in Beispiel 1 beschriebenen Apparatur wurden 1279 g einer 36,95 %igen, aus einer H₂O₂-Oxidation stammenden, wäßrigen Hydroxypivalinsäurelösung vorgelegt und mit 662 ml n-Butylacetat und 500 ml Cyclohexan versetzt. Die Lösung wurde bei Normaldruck bis auf einen H₂O-Restgehalt von 0,14 Gew.-% nach Karl-Fischer-Titration azeotrop entwässert. Während der Entwässerung färbte sich die Lösung gelblich. Die organische Hydroxypivalinsäurelösung wurde schnell bis auf 60°C abgekühlt, dann mit 10°C/h weiter auf -5°C gekühlt und über Nacht bei -5°C gerührt. Die Hydroxypivalinsäurekristalle wurden abgesaugt, zweimal mit je 133 ml des Lösungsmittelgemisches gewaschen und bei 150 mbar/60°C im Trockenschrank getrocknet. Man erhielt 367,2 g Hydroxypivalinsäure mit einem Gehalt von 92,1 % (GC int. Std.) 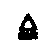 71,6 % der in der Einsatzlösung vorhandenen Hydroxypivalinsäure. Dieses Beispiel zeigt, daß die azeotrope Entwässerung bei Normaldruck auch unter Benutzung eines Lösungsmittelgemisches nicht zur erforderlichen Produktqualität führt.

### Beispiel 3

In der in Beispiel 1 beschriebenen Apparatur wurden 1931 g einer aus einer H₂O₂-Oxidation stammenden, wäßrigen Hydroxypivalinsäurelösung mit einem Hydroxypivalinsäuregehalt von 36,95 % vorgelegt und bei ca. 40°C Kopftemperatur/60 bis 70 mbar 515 g Wasser abdestilliert. Die Destillationseinrichtung wurde durch einen Wasserabscheider ersetzt, und zur Vorlage wurden 1000 g n-Butylacetat und 500 g Cyclohexan gegeben. Bei 160 bis 280 mbar/40 bis 45°C Sumpftemperatur destillierte man weitere 286 g Wasser azeotrop ab. Die Entwässerung wurde bei einem Restwassergehalt von 1 Gew.-% (nach Karl-Fischer-Titration) abgebrochen. Die Hydroxypivalinsäurelösung überführte man in einen 2 l-Doppelmantelsulfierbecher mit Rührer und Innenthermometer, der mit einem Kryostaten temperierbar war. Die Lösung wurde schnell auf 50°C abgekühlt und anschließend in 4 h von 50°C auf -5°C weiter gekühlt. Es wurde eine weitere Stunde bei -5°C gerührt, dann wurde die kristallisierte Hydroxypivalinsäure abgesaugt und zweimal mit je 200 ml auf -5°C gekühltem Lösungsmittelgemisch gewaschen. Nachdem 15 min. Luft durch den Filterkuchen gesaugt worden war, trocknete man die Hydroxypivalinsäure über Nacht im Trockenschrank bei 200 mbar/60°C. Man erhielt 544 g Hydroxypivalinsäure mit einem Gehalt von 98,0 % (GC int. Std.), das entsprach 74,7 % der in der Einsatzlösung vorhandenen Hydroxypivalinsäure.

### Beispiel 4

In der in Beispiel 1 beschriebenen Apparatur wurden 1892 g einer aus einer H₂O₂-Oxidation stammenden, wäßrigen Hydroxypivalinsäurelösung mit einem Hydroxypivalinsäuregehalt von 37,5 Gew.-% vorgelegt, und bei 62 bis 66 mbar/37 bis 45°C Sumpftemperatur wurden 432,4 g Wasser abdestilliert. Anschließend wurde die Destillationseinrichtung durch einen Wasserabscheider ersetzt. Man gab 1285 ml Toluol und 215 ml 1-Butanol zu und destillierte bei 130 bis 300 mbar/40 bis 60°C Sumpftemperatur weitere 418 g Wasser azeotrop ab. Die Lösung hatte dann einen Restwassergehalt von 0,8 % nach Karl-Fischer-Titration. Die Hydroxypivalinsäurelösung überführte man in einen 2 l-Doppelmantelsulfierbecher mit Rührer und Innenthermometer, der mit einem Kryostaten temperierbar war. Die Lösung wurde schnell auf 40°C abgekühlt und dann mit 10°C/h auf -5°C weiter gekühlt. Bei -5°C wurde eine weitere Stunde zur Vervollständigung der Kristallisation gerührt und dann die kristallisierte Hydroxypivalinsäure auf einer gekühlten Glasfilternutsche abgesaugt. Durch den Filterkuchen wurde 10 min. Luft gesaugt, und anschließend wurde zweimal mit je 200 ml -5°C kaltem Lösungsmittelgemisch gewaschen. Die Hydroxypivalinsäurekristalle wurden bei 150 mbar/30°C im Trockenschrank getrocknet. Man erhielt 504 g Hydroxypivalinsäure mit einem Gehalt >99,9 % (GC int. Std.), das entsprach 71,1 % der in der Oxidationslösung vorhandenen Hydroxypivalinsäuremenge; Fp.: 126 bis 127°C.

## Patentansprüche

1. Verfahren zur Isolierung von Hydroxypivalinsäure aus wäßrigen Lösungen durch azeotrop-destillative Entfernung des Wassers und anschließende Kristallisation der Hydroxypivalinsäure aus einem organischen Lösungsmittel, dadurch gekennzeichnet, daß man (a) der wäßrigen Lösung ein oder mehrere organische Lösungsmittel zusetzt, von denen wenigstens eines mit Wasser ein Azeotrop bildet, (b) unter vermindertem Druck soviel Wasser abdestilliert, daß die dabei entstehende Lösung der Hydroxypivalinsäure in dem (den) organischen Lösungsmittel(n) einen Restwassergehalt von mindestens 0,1 Gew.-%, bezogen auf das Gesamtgewicht der enstandenen Lösung, hat und (c) die entstandene Lösung so einstellt, daß sie mindestens ein polares und mindestens ein unpolares Lösungsmittel enthält und die Hydroxypivalinsäure daraus kristallisiert.

2. Verfahren nach Anspruch 1, dadurch gekennzeichnet, daß ein unpolares Lösungsmittel aus der Gruppe der aliphatischen und aromatischen C₆₋C₁₀-Kohlenwasserstoffe, bevorzugt aus der Gruppe von Benzol, Toluol, Xylol, Ethylbenzol, Cyclohexan, n-Hexan, Methylcyclohexan oder ein Gemisch mehrerer von ihnen eingesetzt wird.

3. Verfahren nach Anspruch 1, dadurch gekennzeichnet, daß ein polares Lösungsmittel aus der Gruppe der C₁-C₆-Alkohole, der Ester mit 3 bis 10 Gesamt-C-Atomen, der C₃-C₈-Ketone, der C₄-C₁₀-Ether und der C₃-C₆-Nitrile oder ein Gemisch mehrerer von ihnen, bevorzugt Methanol, Ethanol, n- oder i-Propanol, n- oder i-Butanol, Pentanol, Hexanol, Ethylacetat, Isopropylacetat, i-, n-Butylacetat, 1-Methoxy-2-propylacetat, Methyl-ethylketon, Methyl-isobutylketon, Methyl-tert.-butylether, Diisopropylether, Acetonitril oder ein Gemisch mehrerer von ihnen eingesetzt wird.

4. Verfahren nach Ansprüchen 1 bis 3, dadurch gekennzeichnet, daß im Gemisch aus einem unpolaren und einem polaren Lösungsmittel wenigstens eines ein Azeotrop mit Wasser bildet.

5. Verfahren nach Ansprüchen 1 bis 4, dadurch gekennzeichnet, daß das Gemisch aus einem unpolaren und einem polaren Lösungsmittel aus Toluol und 1-Butanol oder n-Butylacetat und Cyclohexan besteht, bevorzugt ein Gemisch aus 95 bis 40 Vol.-Teilen Toluol und 5 bis 60 Vol.-Teilen 1-Butanol bzw. 80 bis 30 Vol.-Teilen n-Butylacetat und 20 bis 70 Vol.-Teilen Cyclohexan darstellt und besonders bevorzugt ein Gemisch aus 95 bis 75 Vol.-Teilen Toluol und 5 bis 25 Vol.-Teilen 1-Butanol bzw. 75 bis 50 Vol.-Teilen n-Butylacetat und 25 bis 50 Vol.-Teilen Cyclohexan darstellt.

6. Verfahren nach Anspruch 1, dadurch gekennzeichnet, daß die azeotrope Entwässerung bei vermindertem Druck so durchgeführt wird, daß eine Sumpftemperatur von 90°C, bevorzugt von 65°C nicht überschritten wird.

7. Verfahren nach Ansprüchen 1 bis 6, dadurch gekennzeichnet, daß bei der azeotropen Entwässerung ein Restwassergehalt von 0,1 Gew.-% im Sumpf nicht unterschritten wird.

8. Verfahren nach Ansprüchen 1 bis 7, dadurch gekennzeichnet, daß zur Kristallisation der Hydroxypivalinsäure das produkthaltige Lösungsmittelgemisch bis zu -18°C, bevorzugt auf bis zu -8°C abgekühlt wird.

9. Verfahren nach Anspruch 1 bis 8, dadurch gekennzeichnet, daß die durch Filtration isolierten Hydroxypivalinsäurekristalle bei max. 60°C unter vermindertem Druck getrocknet werden.

10. Verfahren nach Anspruch 1, dadurch gekennzeichnet, daß Hydroxypivalinsäure eingesetzt wird, die durch Kondensation von Isobutyraldehyd mit Formaldehyd unter Trialkylaminkatalyse und anschließender H₂O₂-Oxidation erhalten wird.

## Claims

1. Process for isolating hydroxypivalic acid from aqueous solutions by removal of the water by azeotropic distillation and subsequent crystallization of the hydroxypivalic acid from an organic solvent, characterized in that (a) one or more organic solvents, of which at least one forms an azeotrope with water, is/are added to the aqueous solution, (b) the water is distilled off under reduced pressure until the resulting solution of the hydroxypivalic acid in the organic solvent(s) has a residual water content of at least 0.1% by weight, based on the total weight of the solution formed, and (c) the solution formed is adjusted such that it comprises at least one polar and at least one non-polar solvent and the hydroxypivalic acid crystallizes out of it.

2. Process Claim 1, characterized in that a non-polar solvent from the group consisting of aliphatic and aromatic C₆-C₁₀-hydrocarbons, preferably from the group consisting of benzene, toluene, xylene, ethylbenzene, cyclohexane, n-hexane and methylcyclohexane or a mixture of two or more of these, is employed.

3. Process according to Claim 1, characterized in that a polar solvent from the group consisting of C₁-C₆-alcohols, esters having 3 to 10 C atoms in total, C₃-C₈-ketones, C₄-C₁₀-ethers and C₃-C₆-nitriles or a mixture of two or more of these, preferably methanol, ethanol, n- or i-propanol, n- or i-butanol, pentanol, hexanol, ethyl acetate, isopropyl acetate, i- or n-butyl acetate, 1-methoxy-2-propyl acetate, methyl ethyl ketone, methyl isobutyl ketone, methyl tert-butyl ether, diisopropyl ether and acetonitrile or a mixture of two or more of these, is employed.

4. Process according to Claims 1 to 3, characterized in that, in the mixture of a non-polar and a polar solvent, at least one forms an azeotrope with water.

5. Process according to Claims 1 to 4, characterized in that the mixture of a non-polar and a polar solvent comprises toluene and 1-butanol, or n-butyl acetate and cyclohexane, is preferably a mixture of 95 to 40 parts by volume of toluene and 5 to 60 parts by volume of 1-butanol, or 80 to 30 parts by volume of n-butyl acetate and 20 to 70 parts by volume of cyclohexane, and is particularly preferably a mixture of 95 to 75 parts by volume of toluene and 5 to 25 parts by volume of 1-butanol or 75 to 50 parts by volume of n-butyl acetate and 25 to 50 parts by volume of cyclohexane.

6. Process according to Claim 1, characterized in that the azeotropic dehydration is carried out under reduced pressure such that a bottom temperature of 90°C, preferably of 65°C, is not exceeded.

7. Process according to Claims 1 to 6, characterized in that the residual water content in the bottom product does not fall below 0.1 % by weight during the azeotropic dehydration.

8. Process according to Claims 1 to 7, characterized in that the product-containing solvent mixture is cooled down to -18°C, preferably down to -8°C, for crystallization of the hydroxypivalic acid.

9. Process according to Claims 1 to 8, characterized in that the hydroxypivalic acid crystals isolated by filtration are dried at a maximum of 60°C under reduced pressure.

10. Process according to Claim 1, characterized in that hydroxypivalic acid which is obtained by condensation of isobutyraldehyde with formaldehyde, with trialkylamine catalysis and subsequent H₂O₂ oxidation, is employed.

## Revendications

1. Procédé d'isolement d'acide hydroxypivalique à partir de solutions aqueuses, par élimination par distillation azéotropique de l'eau et ensuite, cristallisation de l'acide hydroxypivalique à partir d'un solvant organique, caractérisé en ce que (a) l'on ajoute à la solution aqueuse, un ou plusieurs solvants organiques, dont l'un au moins forme un azéotrope avec l'eau, (b) on distille sous pression réduite suffisamment d'eau pour que la solution d'acide hydroxypivalique dans le ou les solvants organiques, qui se forme, présente une teneur en eau résiduelle d'au moins 0,1% en poids, sur base du poids total de la solution formée, et (c) on ajuste la solution formée, de sorte qu'elle contient au moins un solvant polaire et au moins un solvant non polaire et on recristallise l'acide hydroxypivalique à partir de celle-ci.

2. Procédé suivant la revendication 1, caractérisé en ce que l'on met en oeuvre un solvant non polaire appartenant au groupe des hydrocarbures en C₆-C₁₀ aliphatiques et aromatiques, de préférence au groupe des benzène, toluène, xylène, éthylbenzène, cyclohexane, n-hexane, méthylcyclohexane ou un mélange de plusieurs de ceux-ci.

3. Procédé suivant la revendication 1, caractérisé en ce que l'on met en oeuvre un solvant polaire appartenant au groupe des alcools en C₁-C₆, des esters avec 3 à 10 atomes C au total, des cétones en C₃-C₈, des éthers en C₄-C₁₀, et des nitriles en C₃-C₆ ou un mélange de plusieurs de ceux-ci, de préférence le méthanol, l'éthanol, le n- ou le i-propanol, le n- ou le i-butanol, le pentanol, l'hexanol, l'acétate d'éthyle, l'acétate de propyle, l'acétate de n- ou de i-butyle, l'acétate de 1-méthoxy-2-propyle, la méthyléthylcétone, la méthylisobutylcétone, le méthyl-t-butyléther, le diisopropyléther, l'acétonitrile ou un mélange de plusieurs de ceux-ci.

4. Procédé suivant les revendications 1 à 3, caractérisé en ce que dans le mélange d'un solvant non polaire et d'un solvant polaire, au moins un forme un azéotrope avec l'eau.

5. Procédé suivant les revendications 1 à 4, caractérisé en ce que le mélange d'un solvant non polaire et d'un solvant polaire consiste en le toluène et le 1-butanol ou l'acétate de n-butyle et le cyclohexane, de préférence un mélange de 95 à 40 parties en volume de toluène et 5 à 60 partie en volume de 1-butanol ou 80 à 30 parties en volume d'acétate de n-butyle et 20 à 70 parties en volume de cyclohexane, et de manière particulièrement préférée, un mélange de 95 à 75 parties en volume de toluène et 5 à 25 partie en volume de 1-butanol ou 75 à 50 parties en volume d'acétate de n-butyle et 25 à 50 parties en volume de cyclohexane.

6. Procédé suivant la revendication 1, caractérisé en ce que la déshydratation azéotropique est réalisée à pression réduite, de sorte que l'on ne dépasse pas une température de la masse de 90°C, de préférence de 65°C.

7. Procédé suivant les revendications 1 à 6, caractérisé en ce que lors de la déshydratation azéotropique, on ne dépasse pas une teneur en eau résiduelle de 0,1% en poids dans la masse.

8. Procédé suivant les revendications 1 à 7, caractérisé en ce que pour la cristallisation de l'acide hydroxypivalique, on refroidit le mélange de solvants contenant le produit, jusqu'à -18°C, de préférence jusqu'à -8°C.

9. Procédé suivant les revendications 1 à 8, caractérisé en ce que les cristaux d'acide hydroxypivalique isolés par filtration sont séchés sous pression réduite à au maximum 60°C.

10. Procédé suivant la revendication 1, caractérisé en ce que l'on met en oeuvre un acide hydroxypivalique qui est obtenu par condensation d'isobutyraldéhyde avec du formaldéhyde sous catalyse par trialcoylamine et ensuite, oxydation par H₂O₂.
